# EUROPEAN PATENT APPLICATION

(11) **EP 4 108 776 A1**
(43) Date of publication of application: **28.12.2022**
(21) Application number: 21180902.5
(22) Date of filing: 22.06.2021
(51) Int. Cl.: C12P 7/62, C12N 1/20, C08G 63/06

(54) **PRODUCTION OF BIOPOLYMERS**

(71) Applicant: CO2BioClean GmbH, 65760 Eschborn (DE)
(72) Inventor: Fantinel, Fabiana, 61476 Kronberg (DE); Carfagnini, Alessandro, 47032 Bertinoro, Forlì-Cesena (IT)
(74) Representative: Patentanwälte Gierlich & Pischitzis Partnerschaft mbB

(57) **Abstract**

The present invention discloses a method for producing PHA polymer using bacteria, by using a two-step process. In the first step the bacteria are grown under heterotrophic conditions using an organic substance as carbon source and exponential growth conditions. In a second step the bacteria are then cultivated under autotrophic conditions under an atmosphere of H₂, CO₂ and O₂, wherein the O₂ content is less than 10 % (v/v) and the pressure is more than 1 barg and at least one carbon source is added before and/or during this step. By this the production of PHA with unique properties and at a high rate is possible.

## Description

### FIELD OF THE INVENTION

The present invention relates to a method for producing polyhydroxyalkanoate (PHA) using a wild type bacteria and extracting the produced PHA in an efficient way.

PHA is the general term for a range of diverse biodegradable polymers that consist of polyesters of 3-hydroxyalkanoic acids. These polymers are of interest due to a broad range of applications and the fact that they are completely biodegradable thus offering little or no long term waste issues.

PHAs are generally classified as short chain length PHAs (sclPHAs), medium chain length PHAs (mclPHAs) or long chain length PHAs (lclPHAs), depending upon the number of carbon atoms of the constituting monomers thereof. SclPHA comprises monomers of C₃-C₅, mclPHA comprises monomers of C₆-C₁₄, and lcl-PHA comprises monomers of more than 14 carbons (>C₁₄). This variation in monomer chain length gives rise to different properties in the polymer, with sclPHAs and lclPHAs both having different properties, sclPHAs having a high degree of crystallinity and being usually rigid and brittle, and lclPHAs being sticky and very difficult to handle. The properties of sclPHAs and lclPHAs limit the range of their applications. But since sclPHAs are easier obtainable there is need of sclPHAs with improved properties.

PHA structures can vary in two ways. First, PHAs can vary according to the structure of the pendant groups, which are typically attached to a carbon atom having (R)-stereochemistry. The pendant groups form the side chain of hydroxy alkanoic acid not contributing to the PHA-carbon backbone. Second, PHAs can vary according to the number and types of their repeat units. For example, PHAs can be homopolymers, copolymers, or terpolymers. These variations in PHA structure can cause variations in their physical characteristics. These physical characteristics make PHAs useful for a number of products that may be commercially valuable.

The stereochemistry in the monomers may be only R or S, or monomers of both types may be present. This further influences the properties of the polymer.

The several types of PHAs make PHAs a versatile family of polymers with properties tuneable by molecular design. For example, short chain length scl-PHA are divided into P3HB, commonly simply polyhydroxobutyrate (PHB), P4HB, (valeric acid copolymer) PHBV, PHBH, P3HB4HB, medium chain length mcl-PHA are PHBH (hexanoic acid copolymer), PHBO (octanoic acid copolymer), PHBD (dodecanoic acid copolymer).

The chemical structure of PHAs may be described as a polymeric chain formed by repetitions of the following unit:

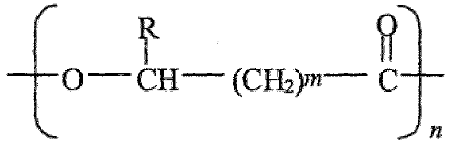

Where R is an alkyl or alkenyl group of variable length and m and n are integers, in some polymers R and m assuming the following values:
PHB: R=CH₃, m=1
PHBV: R=CH₃ or CH₃-CH₂-, m=1
P4HB: R=H, m=2
P3HB4HB: R=H for m=2 or R=CH₃ for m=1

For mclPHA the length of the alkyl chain of R can be different, e.g. for polyhydroxyhexanoate PHBH R is CH₃-CH₂-CH₂- and m equals 2.

Due to their structure the PHA monomer units contain a chiral carbon atom. The polymer may therefore comprise monomers differing in their configuration. PHA synthesized by organisms usually only contains monomers in R-configuration due to the enzymatic pathway.

Besides plants and other organisms, bacteria are very useful for producing PHA. In recent years many efforts were taken to use genetically modified or unmodified bacteria in order to produce PHA also at an industrial scale.

Schlegel et al. Nature 1961, 191, 463-465 "Formation and utilization of poly-β-hydroxybutyric acid by Knallgas bacteria (*hydrogenomonas*)*"* found that PHAs, namely PHB can be produced under certain conditions, especially using an atmosphere comprising CO₂, H₂ and O₂.

Ayaaki Ishizaki and Kenji Tanaka Journal of fermentation and bioengineering 1990, 69(3), 170-174 "Batch Culture of Alcaligenes eutrophus ATCC 17697T using recycled gas closed circuit culture system", Ayaaki Ishizaki and Kenji Tanaka Journal of Fermentation and bioengineering 1991, 71(4) 254-257. "Production of Poly-b-Hydroxybutyric Acid from Carbon Dioxide by Alcaligenes eutrophus ATCC17697T" and Toshihiro Takeshita et al. J. Fac. Agr. Kyushu Univ. 1993, 38(1-2), 55-64. "Studies on Dissolved Hydrogen Behavior in Autotrophic Culture of Alcaligenes autrophus ATCC 17697T" disclose the synthesis of PHB in bacteria under autotrophic conditions. One drawback of the conditions is the requirement of ratio of hydrogen and oxygen, which is explosive (oxygen > 6 %). This limits the use of these conditions.

Kenji Tanaka and Ayaaki Ishizaki Journal of fermentation and bioengineering, 1994, 77(4), 425-427 "Production of Poly-D-3-Hydroxybutyric Acid from Carbon Dioxide by a Two-Stage Culture Method Employing Alcaligenes eutrophus ATCC 17697 T" discloses a two stage heterotrophic-autotrophic growth with fructose and O₂ in autotrophic stage in an amount of 2-3%. But the PHA storage efficiency of bacteria is decreased with organic substrates.

The use of CO₂ as carbon source makes these processes very valuable for the environmentally friendly production of plastics, which are even biodegradable.

US 5,942,597 and WO 97/07229 A1 describe the extraction from PHA from oil plants using solvent mixtures.

Among other PHA types, currently mostly PHB is successfully produced on industrial scale by process that make use of glucose as feedstock. PHB obtained by industrial process are highly crystalline due to their chemical structure composed by a single monomeric unit which is optically pure (high stereoregularity). However, introducing chain irregularities from comonomer, or insertion, e.g. 4B vs. 3B or else stereoregularity is very important to achieve improved flexibility and thus better performances and processability for most general plastics applications.

The procedures presented in the prior art are not suitable for industrial scale processing. Also, the extraction of the PHA produced from the bacteria is difficult. They also require long reaction times.

### BRIEF SUMMARY OF THE INVENTION

It is, therefore, an object of the present invention to provide a method for producing and preferably further purifying PHA in bacteria, more preferably on an industrial scale.

This aim is achieved by the inventions as claimed in the independent claims. Advantageous embodiments are described in the dependent claims.

The object of the invention is also achieved by a method. In what follows, individual steps of a method will be described in more details. The steps do not necessarily have to be performed in the order given in the text. Also, further steps not explicitly stated may be part of the method.

The object of the invention is achieved by a method for producing PHA comprising the following steps:
a) growing bacteria under heterotrophic conditions in a media;
b) cultivating the bacteria under autotrophic conditions under an atmosphere of CO₂, H₂ and optional O₂, wherein the amount on O₂ is below 10 % (v/v) and pressure is at least 1 barg, wherein at least one carbon source is added before and/or during step b).

The bacteria that are useful in the present invention include any bacteria that can produce PHAs, preferably bacteria which naturally produce PHAs. Wild type bacteria are preferred. Such bacteria are not genetically engineered. By using a wild type the process has to fulfil less strict regulations.

In one embodiment, the bacterium is *Pelomonas saccharophila* (also formerly known as *Pseudomonas saccharophila*), *Azomonas lata* (also formerly known as *Alcaligenes latus*) and *R. eutropha* (also known as *Cupriavidus necator*). These are non-pathogenic, gram-negative bacteria, which can be found in soil and water. Their facultative chemolithoautotrophic metabolism allows them to grow either on organic compounds or using H₂ and CO₂ as reductive agent and carbon source, respectively, when submitted to a nutrient limitation and in presence of oxygen. Both modes can also be concomitant depending on the availability of nutrients. These bacteria produce PHB if used in the inventive process without any co-monomer.

In a preferred embodiment the bacteria are the wild bacteria selected from *Cupriavidus necator,* even more preferably stream *Cupriavidus necator* H16, which is a non-pathogenic, gram-negative stream. Other preferred steams are the streams available under the DSM numbers DSM -428, DSM-531, DSM-11098, DSM-3102, DSM-529, DSM-545 at the DSMZ-German Collection of Microorganisms and Cell Cultures GmbH.

Additionally, wild bacteria selected from *Preudosomas Putida* or *Aeuruginosa* can be used.

In the first step the bacteria are grown under heterotrophic conditions. These are conditions utilizing organic compounds as carbon and energy source. In a preferred embodiment under these conditions the bacteria show exponential growth.

In a preferred embodiment the bacteria are grown using conditions with no limitations regarding the nutrients, especially nitrogen, carbon or phosphor.

Usually an ambient atmosphere is used.

In a preferred embodiment the step is performed at ambient pressure or a pressure resulting only from the reaction conditions, e.g. heating in a closed vessel.

The medium used for step a) is an aqueous medium.

In a preferred embodiment the medium for the first step comprises at least one Ammonium salt as nitrogen source, preferably Ammonium sulphate.

As carbon source different organic substances may be used. This may be sugars like sucrose, fructose or glucose, polyols like glycerol, organic acids or salts or esters thereof, as acetic acid or malate or ethyl acetate. The carbon source is preferably soluble in the medium.

In a preferred embodiment the medium for the first step comprises at least one phosphate salt as phosphor source, preferably ammonium, sodium or potassium salts of phosphates, especially in their monobasic form may be used, more preferably an H₂PO₄ salt, more preferably (NH₄)H₂PO₄, KH₂PO₄ and/or NaH₂PO₄. The salts may be used a hydrate.

Ammonium hydroxide, citric acid and/or sulfuric acid may be used for adjusting the pH.

The medium may comprise further salts and additives, like magnesium salts, iron salts, vitamins or trace elements like Zn, B, Co, Cu, Ni, Mo or Mn.

The pH of the medium is preferably 4.5 to 7.5, more preferably 6.4 to 7.1.

In a preferred embodiment the amount of C at the beginning of step a) is between 2 and 50 g/ preferably, between 5 and 20 g/l.

In a preferred embodiment the amount of N at the beginning of step a) is between 0.1 and 5 g/l, preferably 0.1 and 2.5 g/l.

In a preferred embodiment the amount of P at the beginning of step a) is between 0.05 and 5 g/l, preferably 0.1 and 3.5 g/l.

In a preferred embodiment at the beginning of step a) the amount of carbon source is 5 to 50 g/l, preferably 10 to 50 g/l. The values depend on the molar mass of the carbon source and may be adapted to the content of C needed.

In a preferred embodiment a content of 5 to 20 g/l C, 0.1 to 2.5 g/l N and 0.1 to 3.5 g/l P is preferred.

In a preferred embodiment the bacteria are added in an inoculum prepared previously.

For the inoculum a content of 5 to 30 g/l C, 0.1 to 1.5 g/l N and 0.2 to 5 g/l P is preferred.

The values for the beginning of step a) refer to the values after adding the inoculum.

The preferred sources for C, N and/or P for inoculum are the same as mentioned for medium for step a).

In a preferred embodiment at the beginning of step a) the following amounts are present:
Carbon source (Glycerol, Sucrose or glucose or fructose) 15 to 70 g/l, (NH₄)₂SO₄ 1 to 5 g/l, KH₂PO₄ 0.5 to 20 g/l, Citric acid x 1 H₂O 0.1-3 g/l and NaH₂PO₄ 0 to 2 g/l.

Further salts of Mg or Ca may be present. Additionally, a trace element solution is added, comprising Zn, Mn, B, Co, Cu, Ni and Mo.

In a preferred embodiment at the beginning of step a) the reactor is filled at a volume of 5 to 20 % of its total volume.

Step a) is preferably run at a temperature between 20 and 40 °C, more preferably at a temperature between 29 to 35 °C.

It may be necessary to stir the reactor.

During the growth of the bacteria the growth nutrients present are consumed. In a preferred embodiment at least some nutrients are fed into the medium in order to keep these nutrients preferably in the ranges as mentioned above.

The feeding may add to the volume of the medium inside the reactor. Preferably no medium is removed during cultivation.

In a preferred embodiment the feeding solution is added at a rate between 0.1 to 5 % /h calculated from the total volume of the reactor, preferably 0.2 to 1 %. The feeding rate can be adapted based on the content of the feed and/or the cultivation conditions, especially the pressure used.

The feeding solution preferably at least comprises at least one carbon source.

In another embodiment the feeding solution comprises at least one carbon source, at least one nitrogen source.

In another embodiment the feeding solution comprises at least one carbon source, at least one nitrogen source and at least one phosphor source. The preferred sources are the same as mentioned for medium for step a)

In a preferred embodiment the carbon source is identical with the carbon source of the starting conditions.

In a preferred embodiment the feeding solution comprises a carbon content of 100 to 500 g/l, preferably 150 to 300 g/l.

Preferably the feeding solution also comprises a content of N of 1 to 10 g/l, preferably 1 to 5 g/l.

Preferably the feeding solution also comprises a content of P of 0.5 to 15 g/l, preferably 1 to 10 g/l.

Preferably the feed comprises the content of C, N and P as previously mentioned or each in their preferred values.

The feed solution may comprise further salts of Ca and /or Mg, as well as acids like citric acid. The feed may also comprise a trace element solution.

In a preferred embodiment the feed comprises 150 to 300 g/l C, 1 to 5 g/l N, 1 to 10 g/l P.

The preferred sources for C, N and/or P for the feed are the same as mentioned for medium for step a)

In a preferred embodiment the feed comprises the following ingredients:
Carbon source (Glucose or sucrose or glycerol) 150 to 300 g/l of C;
(NH₄)₂SO₄ and/or (NH₄)H₂PO₄ in an amount for 1 to 5 g/l of N;
KH₂PO₄ 0 to 15 g/l;
NaH₂PO₄ 0 to 15 g/l;

The growth of the bacteria is continued until a cell density of at least 10 (measured by OD at 600 nm), preferably at least 15, more preferably at least 20 is reached. The relevant growth point may also be related to other measurements, like time or composition.

Step a) is usually run for at least 5 hours up to 40 hours, preferably 10 hours up to 25 hours.

In the next step the bacteria are grown under autotrophic conditions, except that additionally a carbon source is added before and/or during this step. Under such conditions CO₂ and the carbon source are used as carbon source for the bacteria.

The amount of carbon source added in such a total amount so that the polymer produced will contain carbon from CO₂ and the added carbon source. By this at least a part of the polymer produced will bind the CO₂ from the used atmosphere.

In a preferred embodiment the carbon source is added to an amount for up to 90 mol.-% of the carbon atoms of the polymer produced. These values are the calculated values, this means that if the polymer produced comprises 100 moles of carbon atoms, only 90 moles of carbon source is added during the process. In an even more preferred embodiment, the amount is between 5 mol.% and 90 mol.-%, even more preferred 5 mol.-% and 70 mol.-%, even more preferred 10 mol.-% and 50 mol.-%. In a preferred embodiment the carbon source is added in an amount of 10 mol.-% to 40 mol.-%.

In this step the media is contacted with an atmosphere comprising H₂, CO₂ and optional O₂. In order to minimize the risk of explosion the content of O₂ is less than 10 % (v/v). In a preferred embodiment the content of CO₂ is between 2 % and 40 % (v/v). In a preferred embodiment the content of H₂ is 50 % to 92 % (v/v).

In a preferred embodiment the content of O₂ is less than 8 % (v/v), preferably less than 6 % (v/v), even more preferably less than 5 % (v/v), especially preferred less than 3 % (v/v).

In a preferred embodiment the content of O₂ is less than 8 % (v/v), CO₂ is between 2 % and 40 % (v/v) and H₂ is between 50 % and 92 % (v/v), preferably the content of O₂ is less than 4 % (v/v), CO₂ is between 4 % and 40 % (v/v) and H₂ is between 50 % and 92 % (v/v).

Surprisingly it has been found that the addition of a carbon source in the second step allows to reduce the amount of H₂ required and allows to use a higher amount of CO₂ without affecting the quality of the product. This reduces the amount of hydrogen to be handled within the system and further reduces the risk connected with a high amount of hydrogen gas in a system. Also, less H₂ is consumed during the process, which makes the process cheaper and easier to control.

In a preferred embodiment the amount of H₂ is between 50 % (v/v) and 80 % (v/v), even more preferred between 50 % and 75 % (v/v.

In a preferred embodiment the amount of H₂ is between 50 % (v/v) and 80 % (v/v), while the amount of CO₂ is more than 10 % (v/v), even more preferred the amount of H₂ is between 50 % and 75 % (v/v) while the amount of CO₂ is more than 20 % (v/v). The values adapt if O₂ is also present in the system in the amounts mentioned above.

In a preferred embodiment of the invention the amount of CO₂ is above 20 % (v/v), while the amount of H₂ is less than 80 % (v/v), preferably the amount of CO₂ is above 20 % (v/v), while the amount of H₂ is less than 75 % (v/v).

In a preferred embodiment of the invention the amount of H₂ is between 50 % (v/v) and 80 % (v/v), while the amount of CO₂ is from 20 % to 50 % (v/v), even more preferred the amount of H₂ is between 50 % and 75 % (v/v) while the amount of CO₂ is from 20 % to 50 % (v/v), preferably 20 % to 45 % (v/v). The values adapt if O₂ is also present in the system in the amounts mentioned above. Preferably the range of CO₂ is adapted based on the amount of O₂ present. O₂ is present preferably in an amount less than 5 % (v/v), preferably less than 3 % (v/v). Such an amount is sufficiently low to increase the safety of the process.

In a preferred embodiment the content of nitrogen in the atmosphere is less than 1 % (v/v) if present. Some minor amounts of nitrogen may be carried into the reaction vessel by not degassing the solutions fed into the reactor.

The source for such an atmosphere may be synthesis gas.

If precursors of further monomers added the amount of CO₂ is preferably between CO₂ 1 and 40 % (v/v), preferably 2 % and 30 % (v/v), preferably in the same ranges as described previously.

The ratios mentioned are the ratios present at the beginning of step b). Since the gases are used during the fermentation it may be necessary to adjust the amounts to the previous ranges during the fermentation. In a more preferred embodiment, the ratios are kept within these ranges during step b).

The pressure in step b) is at least 1 barg or gauge pressure. In a preferred embodiment the pressure is at least 2 barg, more preferably at least 3 barg.

In a preferred embodiment the pressure ranges from 2 to 20 barg, preferably 3 to 20 barg, more preferably 3 to 10 barg. The pressure is measured under cultivation conditions.

The pressure is the preferably kept in these ranges during step b). In a preferred embodiment the pressure during whole step b) is at least 1 barg.

Based on the standard atmospheric pressure of 1.013 bar 1 barg as used in the present application corresponds to 2.013 bar absolute pressure. All other ranges are adapted accordingly.

By using increased pressure, the growth of PHA is expedited and the duration of step b) is shortened. Surprisingly the increased pressure also led to PHA with different properties compared to PHA obtained without increased pressure.

Preferably the content of the different gases is measured by the partial pressures.

Preferably the pressure is kept constant for the duration of step b). More preferably the atmosphere composition and pressure are kept constant for the duration of the step b).

It was now found that the quality of the product can be improved if in step b) at least one carbon source is fed into the system. By this the amount of H₂ needed can be reduced, preferably the amount of H₂ and O₂ needed can be reduced.

In a preferred embodiment the carbon source can be the same as described previously. This may be sugars like sucrose, fructose or glucose, polyols like glycerol, organic acids or salts or esters thereof, as acetic acid or malate or ethyl acetate. The carbon source is preferably soluble in the medium. In preferred embodiment the carbon content is in the same range as described for the feeding solution for step a)).

In a preferred embodiment the feeding solution in step b) comprises a carbon content of 100 to 500 g/l, preferably 150 to 300 g/l.

In a preferred embodiment only at least one carbon source is fed in step b). In step b) no further nutrients are fed into the reactor.

The addition of the carbon source can be in different point in time during and/or before step b). It is preferred that it is added after step a).

The carbon source can be added in a separate step before the pressure is applied. It is also possible that it is added during step b). It is also possible that the feeding starts before applying the atmosphere and ends during step b). The addition can be continuous or in one or more portions. It is important that the respective amount of carbon source is added in total.

The addition of the carbon source can be performed in a different reactor or the same reactor of the cultivating step. It is also possible that it is added to a different reactor, which is connected with the cultivating reactor.

In a preferred embodiment the carbon source is added at a rate between 0.1 to 5 % /h calculated from the total volume of the reactor, preferably 0.2 to 1 %. The feeding rate can be adapted based on the content of the feed and/or the cultivation conditions, especially the pressure used.

The pH in step b) is preferably 6.5 to 7.5, more preferably 6.8 to 7.0. The pH may be adjusted using acid and/or bases, preferably sulfuric acid and/or ammonium hydroxide.

As a medium the same medium as step a) may be used, but without any nitrogen source.

In step b) the cultivation is preferably run under at least nitrogen deficient conditions. The nitrogen source limits the biomass accumulation. This leads to PHA accumulation.

In a preferred embodiment the cell dispersion at the end of step a) is directly used for step b) as starting medium.

The temperature in step b) is preferably between 20 °C and 45 °C, more preferably between 25 °C and 35 °C.

It may be necessary to stir the reactor during the reaction.

In a further embodiment of the invention precursors for further monomers are added at step b) in order to obtain copolymers of PHB. In a preferred embodiment these are salts of organic acids, preferably sodium or potassium salts. Examples for such salts are the corresponding salts of propanoic acid, butanoic acid, pentanoic acid or hexanoic acid, depending on the length of the side chain needed. These precursors are usually added in an amount to obtain a unit content of 5 % to 30 % in the PHA produced, preferably 5 % to 20 % (by molar ratio). These precursors are added preferably up to an amount of 1 to 20 g/L, preferably 2 to 10 g/l.

The reaction in step b) is run until the amount of PHA is formed, usually until a content of 50 % to 90 % of PHA by weight calculated from the dry weight of the whole biomass is formed.

Under these conditions it is possible to run step b) until final cell densities of more than 50 g/L, preferably more than 100 g/L is reached.

The reaction is also preferably stopped before the Mw of the PHA and/or PHB starts decreasing due to side reactions.

The duration of the cultivation is usually 20 to 80 hours, preferably 30 to 60 hours, more preferably 30 to 50 hours.

Step b) may be run in the same or a different reactor than step a), preferably a different reactor.

If necessary further purification steps, like filtration or centrifugation are performed between the two steps.

In a preferred embodiment the cells are separated from the medium before the next purification steps.

It is also possible to wash the cells with an alcohol like methanol and/or ethanol.

The PHA is formed inside the bacteria. For the extraction several methods are possible.

In an embodiment the cells are first broken by mechanical stress, e.g. increased atmosphere pressure. The PHA is then extracted using a solvent for PHA, preferably a polar organic solvent, more preferably acetone or chloroform, especially acetone.

In a preferred embodiment a further solvent with a higher boiling point than the solvent for PHA is also added. For example, an oil or an alkane with 10 to 14 carbon atoms. The further solvent is preferably used in a ratio of 1:20 to 1:4 by weight compared to the solvent for PHA.

If from this mixture the solvent for PHA is removed the PHA can be recovered as flakes of high purity. Preferably the solvent for PHA is removed by heat. The solvent for PHA may be reused for a further extraction.

It is also possible that the cell lysis is combined with the extraction step, since acetone also leads to cell lysis. In this embodiment the acetone and optionally the further solvent is added to the separated cells. The amount of solvent for PHA, especially acetone, and further solvent is preferably used in an excess compared to the weight of the separated cells. Preferably in an amount of at least 2 times the weight, more preferably at least 5 times the weight of the separated cells.

In a preferred embodiment solvent for PHA and the further solvent is added and the mixture is then mixed and the nonaqueous phase is separated.

The PHA produced is obtained as flakes during the removal of the solvent for PHA, especially acetone.

The PHA polymer produced by the present process has a narrow molecular weight distribution.

Unexpectedly the PHA polymer as produced is less crystalline polymer than PHA produced under ambient conditions. This makes the for example the PHB polymer less fragile than the typical PHB polymer. They also show a lower modulus than these polymers.

In a preferred embodiment the amorphous content of the polymer is between 25 % to 50 % (measured with solid state NMR).

Usually co-monomers are used to obtain less crystalline polymers, which are usually smoother and more elastic. For example pure PHB produced according to the invention shows some properties similar to PHBV produced by regular procedures and a content of 10 mol.-% co-monomer. Also for PHBV produced with the process of the invention a lesser amount of co-monomer is needed to obtain the same properties.

Another object of the invention is an PHA polymer produced by the process of the present invention.

The polymer can be used for various products depending on its properties. It may also be blended with other polymers.

Another object of the invention is a moulded article, granulate or a master batch comprising or formed from a PHA polymer as described previously.

The moulded articles can thereby be produced in any way, for example by extrusion, casting, injection moulding, pressing, sintering, calendering, film-blowing, melt-spinning, compression moulding and/or thermoforming, for components in automobile construction, transport and/or communications, components for industrial equipment, machine- and plant construction, household appliances, containers, devices for medical technology, components for electrics or electronics. Hence, the invention likewise relates to the use of a polymer material according to the invention for the previously mentioned purposes.

The polymer may be used for the production of coating materials, foils, films, laminates, fibers, moulded parts, moulded articles, injection moulded articles, e.g. bottles or fibers, extrudates, containers, packaging materials, coating materials, particles, beads, micro beads and medicine dispensers.

The polymer can be formed to any product as known from the previous products. It is also possible to add usual additives and other polymers.

### Figures

Fig. 1: Profile fitting of the methylene resonance in the ¹³C CPMAS NMR at 4 ms of a) PHB (commercial) and b) PHB_CO₂ according to the pressure process;
Fig. 2: ¹³C VCT curves of PHB_CO₂ (larger grey dots) and PHB_comm (small black squares) samples.

### Examples

NaH₂PO₄ is used as dihydrate.

### Comparative Example 1A (no additional carbon source in step b))

A seed culture medium A was prepared with Glucose 20 g/l, (NH₄)₂SO₄ 4 g/l, MgSO₄x 4H₂O 1.2 g/l, KH₂PO₄ 4 g/l, Citric acid x 1 H₂O 1.86 g/l and Trace elements solution 10 ml/l (ZnSO₄ x 7 H₂O 0.10 g, MnCl₂ x 4 H₂O 0.03 g, H₃BO₃ 0.30 g, CoCl₂ x 6 H₂O 0.20 g, CuCl₂ x 2 H₂O 0.01 g, NiCl₂ x 6 H₂O 0.02 g, Na₂MoO₄ x 2 H₂O 0.03 g and distilled water 1000.00 ml).

To obtain the inoculum the bacteria (*Cupriavidus necator* H16) is added to the seed culture medium and the inoculum is added to a reactor.

Under growing conditions, a feed solution is added to the reactor. For chemolithotropic growth the following feed solution was used comprising Glucose 660 g/l, (NH₄)₂SO₄ 12 g/l, NaH₂PO₄ 12 g/l, MgSO₄ x 7H₂O 3.6 g/l, KH₂PO₄ 12 g/l, Citric acid 30 g/l, Trace element solution 15 ml/l.

This solution was fed to the reactor under stirring at a rate of 3 - 5 ml/h until an OD of 20 is reached. Usually this is before a reaction time of 21 h.

For autolithotropic growth the reaction mixture is either put into a new reactor or stays in the same reactor.

For autolithotropic growth CO₂, H₂ and O₂ is fed to the reactor with a total pressure of 3 barg (H₂: 80%, 2.4 barg; CO₂: 17 %, 0.5 barg; O₂: 3%, 0.1 barg) .

The reactor is stirred and the fermentation is run until an OD >200 is reached. Usually the reaction time is at least 50 hours, e.g. after 92 hours and OD of 342.67 g/l is reacted in the present example).

The fermentation is then stopped and the PHA (PHB) is extracted.

### Comparative Example 1B (no additional carbon source in step b))

A seed culture medium B was prepared with Sucrose 20 g/l, (NH₄)₂SO₄ 2 g/l, MgSO₄x 4H₂O 1.0 g/l, KH₂PO₄ 0.6 g/l, Citric acid x 1 H₂O 0.11 g/l, NaH₂PO₄ 1.43 g/l, CaCl₂ x 2 H₂O 0.1 g/l and Trace elements solution 3 ml/l (ZnSO₄ x 7 H₂O 0.10 g, MnCl₂ x 4 H₂O 0.03 g, H₃BO₃ 0.30 g, CoCl₂ x 6 H₂O 0.20 g, CuCl₂ x 2 H₂O 0.01 g, NiCl₂ x 6 H₂O 0.02 g, Na₂MoO₄ x 2 H₂O 0.03 g and distilled water 1000.00 ml).

The seed culture medium was inoculated with bacteria (Cu*priavidus necator* H16) and the inoculum is added to a reactor.

Under growing conditions, a feed solution is added to the reactor. For chemolithotropic growth the following feed solution was used comprising Sucrose 600 g/l, (NH₄)₂SO₄ 14 g/l, NaH₂PO₄ 7.69 g/l, MgSO₄ x 7H₂O 4.5 g/l, KH₂PO₄ 2 g/l, Citric acid 0.22 g/l, Trace element solution 15 ml/l.

This solution was fed to the reactor under stirring at a rate of 3 - 5 ml/h until an OD of 20 is reached. Usually this is before a reaction time of 21 h.

For autolithotropic growth CO₂, H₂ and O₂ is fed to the reactor with a total pressure of 3.1 barg (H₂: 81%, 2.5 barg; CO₂: 16 %, 0.5 barg; O₂: 3%, 0.1 barg) . Propionic acid to an amount of 4 g/l in total is added stepwise.

The reactor is stirred and the fermentation is run until an OD >200 is reached. Usually the reaction time is at least 50 hours, e.g. after 53 hours and OD of 220 g/l is reacted in the present example). This leads to 75.2 g/l product. From these cells PHBV is obtained. (Elastic modulus 0.95 GPa, Tm = 167 °C, Content of V approx. 6%, Eta= 4.3 g/1).

### Comparative Example 1C (no additional carbon source in step b))

A seed culture medium B was prepared with Sucrose 20 g/l, (NH₄)₂SO₄ 2 g/l, MgSO₄x 4H₂O 1.0 g/l, KH₂PO₄ 0.6 g/l, Citric acid x 1 H₂O 0.11 g/l, NaH₂PO₄ 1.43 g/l, CaCl₂ x 2 H₂O 0.1 g/l and Trace elements solution 3 ml/l (ZnSO₄ x 7 H₂O 0.10 g, MnCl₂ x 4 H₂O 0.03 g, H₃BO₃ 0.30 g, CoCl₂ x 6 H₂O 0.20 g, CuCl₂ x 2 H₂O 0.01 g, NiCl₂ x 6 H₂O 0.02 g, Na₂MoO₄ x 2 H₂O 0.03 g and distilled water 1000.00 ml).

The seed culture medium was inoculated with bacteria (Cu*priavidus necator* H16) and the inoculum is added to a reactor.

A seed culture medium C was prepared with Glycerol 50 g/l, (NH₄)₂SO₄ 4 g/l, MgSO₄x 4H₂O 1.2 g/l, KH₂PO₄ 13.3 g/l, Citric acid x 1 H₂O 1.85 g/l and Trace elements solution 10 ml/l (ZnSO₄ x 7 H₂O 0.10 g, MnCl₂ x 4 H₂O 0.03 g, H₃BO₃ 0.30 g, CoCl₂ x 6 H₂O 0.20 g, CuCl₂ x 2 H₂O 0.01 g, NiCl₂ x 6 H₂O 0.02 g, Na₂MoO₄ x 2 H₂O 0.03 g and distilled water 1000.00 ml).

To the seed culture medium the bacteria (*Cupriavidus necator* H16) is added to obtain the inoculum and the inoculum is added to a reactor.

Under growing conditions, a feed solution is added to the reactor. For chemolithotropic growth the following feed solution was used comprising Glycerol 500 g/l, (NH₄)₂SO₄ 12 g/l, NaH₂PO₄ 12 g/l, MgSO₄ x 7H₂O 3.6 g/l, KH₂PO₄ 12 g/l and Trace element solution 15 ml/l.

This solution was fed to the reactor under stirring at a rate of 3 - 5 ml/h until an OD of 20 is reached. Usually this is before a reaction time of 21 h.

For autolithotropic growth CO₂, H₂ and O₂ is fed to the reactor with a total pressure of 3.1 barg (H₂: 80.6%, 2.5 barg; CO₂: 16.1 %, 0.5 barg; O₂: 3.2%, 0.1 barg) . Propionic acid to an amount of 6 g/l is added stepwise.

The reactor is stirred and the fermentation is run until an OD >200 is reached. Usually the reaction time is at least 50 hours. From these cells PHBV is obtained.

### Example 1E

A seed culture medium A was prepared with Glucose 20 g/l, (NH₄)₂SO₄ 4 g/l, MgSO₄x 4H₂O 1.2 g/l, KH₂PO₄ 4 g/l, Citric acid x 1 H₂O 1.86 g/l and Trace elements solution 10 ml/l (ZnSO₄ x 7 H₂O 0.10 g, MnCl₂ x 4 H₂O 0.03 g, H₃BO₃ 0.30 g, CoCl₂ x 6 H₂O 0.20 g, CuCl₂ x 2 H₂O 0.01 g, NiCl₂ x 6 H₂O 0.02 g, Na₂MoO₄ x 2 H₂O 0.03 g and distilled water 1000.00 ml).

To obtain the inoculum the bacteria (*Cupriavidus necator DSM 545)* is added to the seed culture medium and the inoculum is added to a reactor. With H16 similar results were obtained.

Under growing conditions, a feed solution is added to the reactor. For chemolithotropic growth the following feed solution was used comprising Glycerol 500 g/l, (NH₄)₂SO₄ 12 g/l, NaH₂PO₄ 12 g/l, MgSO₄ x 7H₂O 3.6 g/l, KH₂PO₄ 12 g/l and Trace element solution 15 ml/l.

This solution was fed to the reactor under stirring at a rate of 3 - 5 ml/h until an OD of 20 is reached at a reaction time of 20 h.

For autolithotropic growth CO₂, H₂ and O₂ is fed to the reactor with a total pressure of 3 barg (H₂: 72%,2.16 barg; CO₂: 0.73 barg 24 %,; O₂: 2%, 0.05 barg)and the following feed solution was used comprising Glycerol 500 g/l is fed with rate of 2 - 5 ml/h. This reduces O₂ content and H₂ consumed. Propionic acid to an amount of 6 g/l is added stepwise.

The reactor is stirred and the fermentation is run until an OD >200 is reached. Usually the reaction time is at least 50 hours, e.g. after 89 hours and OD of 344.17 g/l is reacted in the present example).

The fermentation is then stopped and the PHA (PHBV) is extracted.

### Comparative example 1 D (atmospheric pressure)

Example A was repeated with conditions in phase 2 in analogue to L. Garcia-Gonzalez et al. Catalysis Today 2015, 257, 237-245 "Sustainable autotrophic production of polyhydroxybutyrate (PHB) from CO2 using a two-stage cultivation system". A seed culture medium A was prepared with Glucose 20 g/l, (NH₄)₂SO₄ 4 g/l, MgSO₄x 4H₂O 1.2 g/l, KH₂PO₄ 4 g/l, Citric acid x 1 H₂O 1.86 g/l and Trace elements solution 10 ml/l (ZnSO₄ x 7 H₂O 0.10 g, MnCl₂ x 4 H₂O 0.03 g, H₃BO₃ 0.30 g, CoCl₂ x 6 H₂O 0.20 g, CuCl₂ x 2 H₂O 0.01 g, NiCl₂ x 6 H₂O 0.02 g, Na₂MoO₄ x 2 H₂O 0.03 g and distilled water 1000.00 ml).

To obtain the inoculum the bacteria (*Cupriavidus necator* H16) is added to the seed culture medium and the inoculum is added to a reactor.

Under growing conditions, a feed solution is added to the reactor. For chemolithotropic growth the following feed solution was used comprising Glucose 660 g/l, (NH₄)₂SO₄ 12 g/l, NaH₂PO₄ 12 g/l, MgSO₄ x 7H₂O 3.6 g/l, KH₂PO₄ 12 g/l, Citric acid 30 g/l and Trace element solution 15 ml/l.

This solution was fed to the reactor under stirring at a rate of 3 - 5 ml/h until an OD of 20 is reached. Usually this is before a reaction time of 21 h.

For autolithotropic growth the reaction mixture is either put into a new reactor or stays in the same reactor.

For autolithotropic growth CO₂, H₂ and O₂ is fed to the reactor at atmospheric pressure with composition of the gas mixture H₂: 84%, CO₂: 13 %, O₂: 3%.

The reactor is stirred and the fermentation is run until an OD >200 is reached. Usually the reaction time is at least 150 hours, e.g. after 184 hours and OD of 230g/l is reached in the present example.

The fermentation is then stopped and the PHB is extracted.

### Material properties

The PHB produced by the described process shows similar thermal properties of PHB produced by standard chemical methods. The molecular weight is in the rage of commercially available PHBs with a narrow MWD. But the polymer is less fragile than typical PHB and more amorphous. The PHB has better tensile properties than pure PBH (elongation brake >20%), a lower modulus (approx. 1000 MPa, vs >2000 MPa), better transparency and a low T_{g} (-8 °C). PHB produced under similar conditions but without increased pressure is crystalline and similar to commercial PHB.

Table 1 compares some properties of the PHB of the invention with a PHB Reference and PHBH produced by a biological process:

**Table 1:**

| Property | PHB | PHBV comp. Example 1C | PHB reference | PHBV Example 1E | PHBH reference | PHB comparative 1D |
|---|---|---|---|---|---|---|
| Density [g/cm³] | 1.25 | 1.20 | 1.25 | 1.2 | 1.2 | 1.25 |
| Molecular weight [g/mol] | 745 k | 712 k | 398 k | 689 k | 500k | 571 k |
| Melting point [°C] | 181 | 167 | 179 | 165 | 145 | 179 |
| Glass T T_{g} [°C] | -8 | -12 | 0 | -10 | 2 | 0 |
| Elongation break [%] | 22 | 150 | 3 | 154 | 14 | 5 |
| Tensile modulus [MPa] | 1010 | 950 | 2500 | 937 | 1350 | 2450 |
| Flexural modulus [MPa] | 1300 | 1230 | 2800 | 1190 | 1600 | 2780 |
| Tensile strength [MPa] | 33 | 30 | 35 | 28 | 36 | 34 |

Although the melting temperature is the same as expected for pure PHB, the modulus and the tensile properties are more similar to those of the PHBH copolymers.

### DSC measurements

Mettler DSC 30 scanner was used. The tests were conducted with a controlled flow of nitrogen. The samples were subjected to the following thermal cycle: two heating steps from -100 °C to 200 °C interspersed by a cooling step from 200 °C to -100 °C. The heating/cooling rate was 10 °C/min.

From the DSC thermograms the melting point (Tₘ₁) of the polymer, the crystallization temperature (T_{c}) under cooling conditions and the melting point in the second heating scan (Tₘ₂) were identified. The integration of the peaks allowed to estimate the melting enthalpy under the first (ΔHₘ₁) and the second (ΔHₘ₂) heating scans and under cooling (ΔH_{c}) conditions.

### Molecular weight

The molecular weight was measured indirectly, by intrinsic viscosity, where relation between the viscosity and the molecular weight is given by the Mark- Houwink expression (a = 0.78, k = 0.000118).

### Nuclear magnetic resonance (NMR)

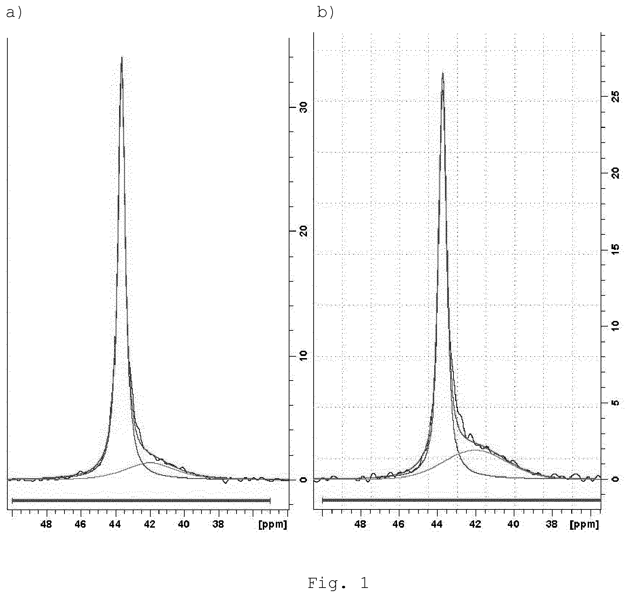

The identification of the PHA's powders was performed through a solid state NMR analysis (¹³C CPMAS NMR, Fig. 1). This were carried out with a Bruker 400WB spectrometer operating at a proton frequency of 400.13 MHz. NMR spectra were acquired with cp pulse sequences under the following conditions: ¹³C frequency: 100.48 MHz, n/2 pulse 3.5 µs, decoupling length 5.9 µs, recycle delay: 4 s, 128 scans; contact time 2 ms. Sample was packed in 4 mm zirconia rotor and spun at 10 kHz under air flow. Adamantane was used as external secondary reference. The spectrum of PHB from CO₂ is superimposable to the one of commercial PHB.

In the NMR spectrum both methyl and methylene signals are represented by sharp peaks together with a right broad shoulder. Thus, these shoulders are proof of a different chain packing in the solid state. The presence of this type of shoulder in the case of other polymers is usually attributed to an amorphous component. The superimposition of the spectra of the two samples highlights a very small difference in the intensity of the above-discussed shoulders. The amorphous component is higher in the PHB sample from the pressure process but without feeding extra carbon source.

Further Experiments of NMR dynamics, measuring magnetization as function of contact time show higher amorphous content 35.9% vs 21.8% expected from the reference PHB sample (profile fitting at 42 ppm and 43.6 ppm). This shows that the packing of the polymer chains is to be different in the PHB produced by the invention. Figure 1 shows the profile fitting of the methylene resonance in the ¹³C-CPMAS NMR at 4 ms of a) PHB reference and b) PHB made by pressure process.

For all samples the PHB according to the invention showed a higher amorphous content compared to the commercial PHB or the sample from the comparative example 1 D (Table 2).

**Table 2**

| | A% | A% | A% | A% | Attrib. |
|---|---|---|---|---|---|
| δ (ppm) | PHB_CO₂ | PHB_referenceC | Comp. Example 1 D | PHBV Example 1E | |
| 43.6 | 64.1 | 78.2 | 76.1 | 75.4 | Crystalline |
| 42 | 35.9 | 21.8 | 23.9 | 24.6 | Amorphous |

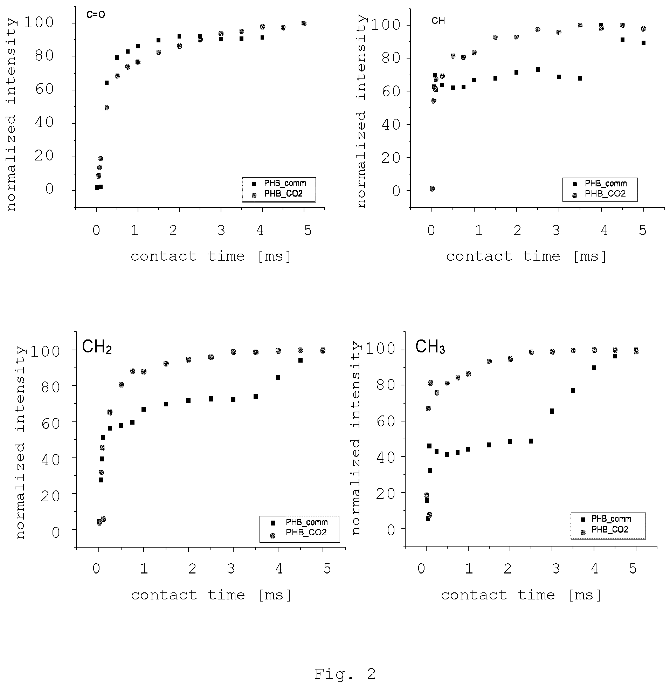

Finally, evaluating the trend of the magnetization (peak area) as a function of contact time (Figure 2) it is possible to relate the behaviour to chain mobility at the molecular level. The normalized curves for the four resonances (C=O (top left), CH (top right), CH₂ (bottom left), CH₃ (bottom right)) are presented in Figure 2: PHB_CO₂ shows a homogeneous trend; instead the PHB_comm seems composed of multiple domains with different mobility. The lower amorphous content of example 1E may be caused by the additional monomer added.

For both materials, the magnetization increases fast as for rigid materials reaching a plateau that suggest a very long decay typical of polymers. The CO region does not show remarkable difference between the two samples. The second step of growth suggests the presence of a second very mobile component not homogeneously distributed. One could hypothesize that the two samples are a different mixture of enantiomers. It is expected that this is an effect of the CO₂ in combination with the increased pressure.

### Uniaxial tensile tests

The test was performed using an Instron tensile tester model 4250 equipped with a 100 N load cell. The test was carried out at a cross-head speed equal to 1 mm/min. The specimens for the test have been prepared by cutting a film of the studied PHB. The film was obtained from the dissolution of the polymer with chloroform into a Petri dish and the subsequent evaporation of the solvent. Five specimens were tested.

## Claims

1. Method for producing PHA comprising the following steps:
a) growing bacteria under heterotrophic conditions in a media;
b) cultivating the bacteria under autotrophic conditions under an atmosphere of CO₂, H₂ and optional O₂, wherein the amount of O₂ if present is less than 10 % (v/v) and pressure is at least 1 barg, wherein at least one carbon source is added before and/or during step b).

2. Method according to claim 1, wherein the bacterium is a wild type bacterium.

3. Method according to one of the claims 1 or 2, wherein the bacterium is *Cupriavidus necator.*

4. Method according to one of the claims 1 to 3, wherein the carbon source is selected from the group of sugars, polyols or organic acids or salts or esters thereof.

5. Method according to one of the claims 1 to 4, wherein in step a) the bacteria are grown under exponential growth conditions.

6. Method according to one of the claims 1 to 5, wherein the pressure in step b) is at least 2 barg.

7. Method according to one of the claims 1 to 6, wherein the pressure in step b) ranges from 2 to 20 barg.

8. Method according to one of the claims 1 to 7, wherein content of CO₂ in step b) is between 2 % and 90 % (v/v).

9. Method according to one of the claims 1 to 8, wherein the content of H₂ in step b) is between 50 % and 92 % (v/v).

10. Method according to claim 9, wherein the content of H₂ in step b) is between 50 % and 80 % (v/v).

11. Method according to claim 10, wherein the content of H₂ in step b) is between 50 % and 75 % (v/v).

12. PHA polymer as produced by the process according to one of the claims 1 to 11.

13. Moulded article, granulate or master batch comprising the PHA polymer according to claim 12.

14. Use of the PHA polymer according to claim 13 for the production of coating materials, foils, films, laminates, fibers, moulded parts, moulded articles, injection moulded articles, extrudates, containers, packaging materials, coating materials, particles, beads, micro beads and medicine dispensers.
